# EUROPEAN PATENT APPLICATION

(11) **EP 1 133 926 A1**
(43) Date of publication of application: **19.09.2001**
(21) Application number: 00302203.5
(22) Date of filing: 17.03.2000
(51) Int. Cl.: A23J 1/00, A23J 3/22, A23J 3/20, C12N 1/14, A23L 1/28

(54) **Foodstuffs containing mucorales fungi**

(71) Applicant: DSM N.V., 6411 TE Heerlen (NL)
(72) Inventor: Bijl, Hendrik Louis, 3131 ZD Vlaardingen (NL); Kruyssen, Fredericus Johannes, 2341 JK Oegstgeest (NL)
(74) Representative: Wright, Simon Mark

(57) **Abstract**

The preparation of an edible substance for use in a foodstuff is described which comprises fungal cells of the order *Mucorales.* The cells are grown in a fermentor vessel in an aqueous liquid which is mixed during fermentation, and fermentation allowed to finish. The fungal cells are kept in the vessel and allowed to ripen, the cells own RNAses reducing the intracellular content. Fungi of this order inherently have a low RNA content and therefore no heating step, to reduce their RNA content, is necessary. The fungal cells are removed and processed into the edible substance. This substance is then processed into a foodstuff. This avoidance of heat has also resulted in better tasting foodstuffs.

## Description

### Field of the Invention

The present invention relates to the preparation of edible (proteinaceous) substances using fungal cells of the order *Mucorales* and the use of these substances in foodstuffs, in particular as meat substitutes.

### Introduction

Animal meat is considered to be a desirable part of the human diet, not only due to the vitamins and nutrients it provides, but also due to its flavour (particularly on cooking) and, importantly, its texture. However, an increasing number of people are turning to vegetarian or vegan diets, neither of which can include meat or meat derived products. Such diets may be due to a number of factors, but is often due to either a disliking for meat (either in texture or flavour) or due to ethical and moral considerations (for example, a belief that it is wrong to kill animals in order to feed humans).

Apart from certain edible fungi (e.g. mushrooms) proteinaceous foods containing fungi are known. One example is the traditional Indonesian fermented food, tempeh. This is usually prepared by the fermentation of *Rhizopus* fungi on soy beans (and parts thereof) acting as a moist solid substrate. The beans (or other vegetable substrate) are inoculated with the fungus and fermentation allowed for 24 to 36 hours. The beans became bound by the fungal mycelium protein produced to give a firm product which can then be sliced before eating (no additional processing is usually performed before consumption). However, apart from lacking much taste or flavour, tempeh is relatively dry and does not have the fibrous and juicy texture associated with meat.

Thus a number of edible meat substitutes or meat replacers have been proposed in recent years. Soy-based products, in particular extruded soy, are marketed, especially by American and Japanese companies, but these do not have a particularly meat-like taste or texture (indeed both soy and gluten can both have an "off' or astringent taste). GB-A-2007077 (Maclennan/BioEnterprises) proposes a similar process to the manufacture of tempeh, except instead of soy beans the solid substrate is a starch-containing food such as sago, cereals or potatoes.

More recently workers have proposed the production of edible protein-containing substances using the production of mycelial protein by the fungus *Fusarium graminearum* (EP-A-0123434). This fungus has been increasingly used in meat substitute products sold in the UK and other European countries. One of the problems, however, with this fungus is that its content of RNA is normally too high for human consumption. Steps must therefore be taken to reduce the RNA content of the fungal cells before they can be included into a foodstuff that can be marketed.

The art is replete with different methods for reducing RNA content. GB-A-2191504 teaches that nucleic acid is metabolised by humans but breaks down to form uric acid and this can cause gout. It therefore suggests first treating microbial cells with a base (pH 7 to 10) and heat (65 to 99°C), then treating the cells with acid (pH 3 to 4.5) and heat (75 to 95°C), followed by heat-shocking the cells (100 to 150°C).

EP-A-0050831 refers to production of nucleic acid reduced protein (NARP) for human consumption by contacting the cells with a mineral acid at a temperature of 50 to 100°C. US 3968009 refers to processes for reducing the nucleic acid content of yeast and bacteria using a heat-shock treatment and GB 1440642 describes a process for decreasing the nucleic acid level in *Fusarium graminearum* by placing the fungus in a suspension at a pH of from 4.7 to 7.0 at a temperature of between 55 and 72°C for a time of at least 60 seconds. GB 1408845 describes a similar process except here ethanol, methanol or propanol is additionally used and the pH is 5 to 9.5, the temperature is 30 to 80°C, and the time period is at least 90 seconds.

The unpublished International application PCT/EP99/07722 describes *Mucorales* fungi for use in foodstuffs but, in keeping with the prior art, teaches that the RNA content of the cells must be reduced for example by heating at a temperature of 40 to 80°C.

Not only is this additional RNA reduction step undesireable, because it adds to costs (because of heating, and often with additional acid or alkali) but this type of treatment has been found to adversely affect the taste of the foodstuff produced. There is therefore a need for a way of incorporating a fungus into a foodstuff, for example as a meat substitute, that may avoid, or at least partially eliminate the need for, such a heating step (either with or without concominant use of acid or alkali). Furthermore, and in any event, it would also be useful to be able to improve the taste of the resulting foodstuff.

### Brief Description of the Invention

According to a first aspect of the invention there is provided a process for the preparation of an edible (e.g. proteinaceous) product comprising fungal cells, that is suitable for use in a foodstuff, the process comprising:
(a) fermenting fungal cells of the order *Mucorales* in an aqueous liquid contained in a fermenter vessel, the liquid comprising an assimilable nitrogen (N) source and an assimilable carbon (C) source, and mixing the liquid and cells in that vessel during fermentation;
(b) separating at least some of the cells from the mixture of fungal cells and aqueous liquid or reducing the liquid content of the mixture of liquid and cells; and
(c) processing the fungal cells into an edible product.

Suitably before (c) the temperature of the fungal cells is kept below 40°C. Also, preferably before (c), the fungal cells are subjected to conditions that allow enzymes inside the fungal cells to reduce the RNA content of the fungal cells.

Although *Fusarium graminearum* has been used in foodstuffs for a number of years now, one of the problems associated with its use is its inherently high RNA content, and this is why there are numerous patent publications that concern different methods for reducing the RNA content. In order to address this problem, instead of investigating how the RNA content of *Fusarium* could be reduced (an approach taken in the art), it was decided to look for alternative fungi that could be used instead of *Fusarium.* The invention is thus partly based on the finding that fungi of the order *Mucorales* have an inherently low RNA content, or that they can be conditioned to reduce their RNA content. As a result, no specific RNA reduction step may be necessary and so one can avoid a (e.g. physical and/or chemical) RNA reduction step that is taught in the art to be essential. The invention may thus avoid RNA reduction by heat or other physical treatment, or by treatment of the cells with chemical(s) and/or enzyme(s)). In other words, one can avoid the (e.g. RNA reducing) heating step and/or chemically degrading the RNA inside the cells. This may not only result in a cheaper process, but unexpectedly the absence of heating was found to improve the taste of the resulting foodstuff. Heating can make the cells more porous (so one can lose valuable compounds) and, without care, can result in lysis of the cells. The process may hence exclude chemically degrading (e.g. by hydrolysis, such as with acid and/or alkali) RNA inside the cells.

Thus it has been found that it is not necessary to heat (or chemically treat) fungal cells of the order *Mucorales* to meet legal requirements because of their inherently low RNA content. Thus in the quest to improve taste, it has been found that it is no longer necessary to include a heat-shock or heat RNA reduction step as good tasting products can be obtained using *Mucorales* fungal cells.

The process can therefore exclude heating the fungal cells (such as to above 40°C) to reduce their RNA content. This step is not necessary because the fungi of the order *Mucorales* have unexpectedly been found to have an inherently low RNA content, in particular if the cells are subjected to conditions that allows the cells to reduce their own RNA content. Although fungi other than *Fusarium* have been suggested as meat substitutes recently (see unpublished International application no. PCT/EP99/07722, *supra),* this followed the trend in the art that advocated heat treatment of fungal cells to reduce their RNA content. This application refers to the use of fungi of the order *Mucorales* in foodstuffs and, due to the (legal) requirement that cells to be used in foodstuffs must have a low RNA content, advocates heat treatment (to reduce RNA) at a temperature of 40 to 80°C. It was thought at that time that if a lower temperature was employed this would not activate enzymes inside the cells that would reduce the RNA content. However, since then it has been unexpectedly found that the RNAses inside the cells are still active below 40°C and can act to reduce the RNA content.

Thus it is preferred that in the process of the present invention, at least before stage (c), the temperature of the fungal cells throughout the process never exceeds, or is always below, 40°C. The fungal cells are therefore subjected to conditions that allow the RNAses inside the fungal cells to reduce their intracellular RNA content. This is preferably achieved after fermentation in stage (a), for example before stage (b).

The *Fusarium* prior art also advocates heating because the cells need to be killed and also because proteases, which would otherwise degrade valuable protein, need to be inactivated.

The fungal cells need to be killed before the final foodstuff is consumed. If this is to be achieved by heating the fungal cells, then the present invention will only require one heat step (for example during processing), whereas in prior art processes two heat steps are required, a first heating step to reduce the RNA content (for example a heat shock treatment) and then a further heating step during foodstuff manufacture (for example, to gel edible protein components that may be added to the cells).

As will be appreciated, the process of the first aspect is a liquid fermentation, in other words the aqueous liquid (e.g. a solution) serves as the culture medium. This contrasts with prior art processes which culture fungi on a solid substrate, that substrate being, for example, edible and/or vegetable in origin such as rice, soy bean or starch-containing products such as cereals or potatoes. In the invention the liquid fermentation process in (a) is preferably conducted in the absence of a solid substrate, such as one which is itself an edible foodstuff (this includes not only vegetable material or legumes but also meat and natural solid starch or carbohydrate-containing substances such as cereals, soybeans, sesame seeds or meal).

Following stage (b) one often obtains a biomass or a filter cake, or such a biomass or filter cake which has been milled, tumbled and/or centrifuged.

Thus a second aspect of the present invention relates to an edible product suitable for use in a foodstuff, preferably preparable by a process of the first aspect. The cells may be dead (non-viable) fungal cells but preferably at this stage they are alive (viable). The product suitably comprises of the order *Mucorales* having an RNA content below 2%. In keeping with the first aspect it is preferred that the cells have been subjected to conditions that allow them to have (inherently, or to have obtained) this maximum 2% RNA content.

The fungal cells will preferably remain intact (or whole) not only during the fermentation process, but also during subsequent processing steps, including water removal and any texturing. Thus the product will contain intact (e.g. non-lysed) fungal cells, and during most if not all stages of the preparation of the substance or the product, steps will be taken to minimize damage to and lysis of the cells or cell membranes. However, during processing some compounds may leave the cell (so that the cell membrane may "leak" a little). However, preferably there is no removal of RNA (or protein) from the fungal cells, either by chemical or physical methods.

The product includes fungal protein produced by the fungal cells. Usually this protein is intracellular and/or within the cell membrane. Extracellular protein may be present but this can be removed if necessary, during processing (e.g. water removal, since the extracellular protein may be present in the aqueous liquid).

The product preferably has at least 40%, e.g. at least 50% or even 60% or more of fungal cells. However these amounts can be much larger and the cells can constitute at least 70%, such as at least 80%, and optimally at least 90% or 95% of the product (on a dry matter weight basis). With such a high content of fungal cells one can obtain a product (and also a foodstuff) that is more juicy, fibrous and better tasting. These percentages are based on the weight of the cells in the dry matter, in other words one firstly dries the substance or product and, on the basis of the dry matter obtained, calculate (by weight) the percentage of that matter that is fungal cells.

The product may consist essentially only of the fungal cells (which can include the protein produced by those cells). During fermentation, therefore, there will usually be no extraction of isolation of any particular compound(s) or substance(s) either contained in or produced by the fungal cells. Any extracellular protein that is produced may be washed away from the cells and so may not be present.

The product of the second aspect is edible in the sense that it can be included into a foodstuff, or it is compatible with food or feed use. Although the product may be eaten as such (for example if the cells are not alive), the intention is that this is in fact an intermediate in the preparation of the foodstuff.

The product thus may be suitable for inclusion into a foodstuff (it can be edible, and suitably digestible, by either humans or animals). The product may be or comprise pellets, granules or sheets, it may be prepared by a process involving extrusion (and therefore may be an extrudate), may comprise a dough, a paste, or a (e.g. meat-like) chunk, or may be in the form of a roll (such as by rolling a sheet).

After the fermentation in (a) the only edible material in the product may be the fungal cells and so this may, apart from the fungal cells, be devoid of edible substances. However, various edible component(s) can then be mixed with or added to the cells to obtain the product. Further or additional edible component(s) may be added to the product in the preparation of the foodstuff.

A third aspect of the present invention relates to a process for the preparation of a foodstuff, which comprises forming a foodstuff with, or including into an existing foodstuff, a product according to the second aspect (for example preparable by a process of the first aspect). This may involve adding one or more additional edible components to the product and/or it may comprise (e.g. further) processing, such as texturization. This process therefore includes not only preparation of a foodstuff using the product, but it also envisages the supplementation of a foodstuff with the product.

A fourth aspect of the present invention is thus provided by the foodstuff, and this can be preparable by process of the third aspect.

The foodstuff may be a sausage, pâté, burger, spread, animal feed or it may include edible pharmaceutical compositions such as tablets.

The foodstuff preferably comprises at least 5%, for example at least 8 or 10%, and optimally at least 15 or 20% of fungal cells (on a dry matter weight basis). The fungal cell content may be as high as that described for the product, but since the foodstuff can be made from that product the fungal content is often lower, for example the fungal cells may constitute only at least 25 or 30% of the foodstuff (again, based on a dry matter weight calculation).

### Detailed Description of the Invention

### Choice of fungi - Murorales

By using (non-toxic) *Mucorales* fungi (e.g. those used in Asian fermented food products) one can also avoid any mycotoxins that may be produced by other (e.g. prior art) organisms. Thus little or no screening for organisms that are safe for inclusion into foodstuffs may be required. This means that the products produced by the invention may be more suitable for ingestion and for use in foodstuffs. The *Mucorales* organisms in general do not produce mycotoxins (or at least an insignificant amount or below detection levels), which is clearly advantageous as these organisms are incorporated whole into a foodstuff, and this can mean that the processing techniques can be more efficient as mycotoxins may not need to be removed. This can overcome a problem with *Fusarium* organisms which can produce undesirable mycotoxins. Mycotoxins anticipated here are those such as aflatoxine, mevinolin, terrein and trichothecenes (the latter being produced by only some *Fusarium* species). A fungus that produces any of these mycotoxins is unlikely to be allowed to be used in any form of food production, even if the manufacturer takes steps to remove the mycotoxins. It is therefore particularly important to choose fungi that will not produce these mycotoxins at any stage of the process.

A further advantage of using *Mucorales* fungi is that a relatively wide variety of microorganisms is available, depending upon the characteristics desired in the proteinaceous substance. This can allow differing physical characteristics (such as in the fibrous nature, or mouthfeel) or in chemical characteristics (taste, etc). The fungi used in the present invention have been found to give improved meat-like properties, for example a more fibrous and/or juicy texture. These fungi can also vary in terms of texture and juiciness and so allow themselves to be used in a wide variety of foodstuffs. Therefore by choice of microorganism one can provide the various desirable characteristics according to the eventual foodstuff to be prepared (using different processing techniques). In addition different microorganisms can impart different colours, and so as well as being able to prepare a white substance, one can make substances that have different colours, such as yellow to brown or even green appearance, which may be desirable for some foodstuffs.

The fungi can be of the family *Choanephoraceae*, such as of the genus *Blakeslea or Gilbertella*, for example of the species *Blakeslea trispora* or *Gilbertella persicaria*. The other three families included within the order *Mucorales* are *Cunninghamellaceae*, *Mortierellaceae* (such as fungi of the genus *Mortierella*, and in particular the species *Mortierella alpina*) and, especially, *Mucoraceae*.

The fungal cells are preferably of the genus *Rhizopus*, *Rhizomucor*, *Mucor* or *Mortierella*, all of which belong to the family *Mucorales*. Suitable fungi of the genus *Rhizopus*, *Mucor* or *Rhizomucor* include *Rhizopus stolonifer*, *Rhizopus miehei*, *Rhizopus pusillus*, *Rhizopus oligosporus* and, in particular, *Rhizopus oryzae*; *Mucor hiemalis* and *Mucor rouxii*; and *Rhizomucor meihei*. Other preferred strains include those of the genus *Absidia* or *Phycomyces,* such as *Absidia pseudocylindrospora* or *Phycomyces blakesleeanus.* Suitable fungi are usually edible (and digestible) by humans or animals.

Preferred fungi can have a cell wall comprising, or primarily containing, chitin and chitosan. The cell walls may contain one or more of the sugars glucosamine (such as D-glucosamine) and/or fucose, such as L-fucose, and may be substantially free of galactose.

The fungi used in the present invention preferably do not have septa, which is in contrast to those of the group *Fusarium*. Furthermore, preferred fungi for use in the invention have branching, again unlike *Fusarium* organisms which have little or no branching (in their hyphae). Indeed, the art advocates the use of non-branching mutants (EP-A-0,123,434). The hyphae of the fungi used in the invention may have a diameter from 1 to 20µm, such as from 2 to 10 µm, optimally from 2 to 8µm.

The fungus may be a naturally occurring one, it may have been selected using known techniques for particular desired properties, or it may be genetically engineered.

Preferred fungi are also of the group *perfecti* (in other words, not belonging to the class *imperfecti*) which are able to reproduce sexually. Fungi used in the invention can also be filamentous.

### Fermentation - Stage (a)

The fermentation process of the first aspect is suitably conducted in a fermenter vessel adapted for containing the aqueous liquid, such as a vat. This vessel may be pressurized. It may be also be adapted to allow the continuous or continual supply of the assimilable nitrogen and/or carbon sources. Stage (a) and later stages are therefore preferably conducted aseptically. Although the fermentation can be a continuous process, with regular harvesting or removal of the fungal cells (and accompanying protein), the process can be a batch process if desired, such as a repeat fed batch process (one or more additions of C and/or sources after fermentation has begun). Thus the fermentation process can be stopped or halted, and the fungal cells removed from the vessel, before another or fresh fermentation is begun.

The carbon and nitrogen sources may be provided in separate compositions. This because the different sources may be subject to different sterilizing conditions, and furthermore it allows a variation in the relative amounts of carbon and nitrogen during fermentation.

The nitrogen and/or carbon sources can be supplied (or added) separately, or supplied simultaneously, or supplied as a combined preparation. They may thus present in the same composition (if thought necessary) which is preferably a liquid. The C and/or N sources can be added (to the fermenter vessel) either before the fungal cells are added (to the vessel), in other words prior to inoculation, or during fermentation.

If the supply is continual (or intermittent), it is preferred that for each instance of supply (e.g. "shots" or additions) the amount of both carbon and/or nitrogen sources is the same.

Preferred C:N (weight) ratios are at least 6:1, but may vary from 10:1 to 150:1, such as from 15:1 to 50:1, optimally from 25:1 to 40:1.

For continual supply, preferably the time during which the nitrogen and/or carbon sources are supplied are greater than the time when they are not. Thus, during fermentation supply is advantageous for at least 50% of the time. If supply of one or both sources is intermittent, then there should be at least 2, preferably at least 5, and optimally at least 10, additions to the aqueous liquid of the nitrogen and/or carbon source. For continuous supply or further additions it is preferred that the C:N ratio in the sources is kept at (about) the same ratio as when fermentation started.

The carbon and/or nitrogen sources may be complex sources, defined media or individual or isolated compounds. Non-complex sources are preferred (these may have or produce fewer mycotoxins) and so the compounds may be added in a high degree of purity, and can be common (or commercially available) chemicals. Preferably both C and/or N sources are not solid, and suitably both are liquids. Preferred nitrogen and/or carbon sources are water soluble or water miscible.

Suitable nitrogen sources include ammonia or ammonium ions. The advantage here is. that ammonia can act as a pH regulant. This may be supplied in the form of an ammonium salt, such as nitrate, sulphate or phosphate or in the form of ammonium ions themselves, for example an aqueous solution of ammonium hydroxide. Other inorganic nitrogen sources can also be used, such as sodium nitrate, urea or an amino acid such as asparagine or glutamine.

Complex nitrogen sources include yeast hydrolysates, primary yeast, soy bean meal, hydrolysates of casein, yeast, yeast extract or rice bran.

The carbon source can comprise (complex sources such as) maltodextrin, oat flour, oat meal, molasses, vegetable (e.g. soy bean) oil, malt extract or starch. Preferred (non-complex) carbon sources include carbohydrates or sugars, such as fructose, maltose, sucrose, xylose, mannitol, glucose, lactose, citrate, acetate, glycerol or ethanol.

The aqueous liquid may additionally contain other substances to assist in the fermentation, for example a chelating agent (e.g. citric acid), an anti-foaming agent (e.g. soy bean oil), a vitamin (e.g. thiamine and/or riboflavin), any necessary catalytic metals (for example, alkali earth metals such as magnesium or calcium, or zinc or iron and/or other metals such as cobalt and copper), phosphorus (e.g. phosphate) and/or sulphur (e.g. sulphate). Preferably the aqueous liquid will have a low sulphur content, for example less than 3.0g, preferably less than 2.0g or 1.0g, of sulphur by litre of aqueous liquid.

Preferably, the pH, temperature and/or oxygen content (of the aqueous liquid) during fermentation is controlled. This may be to keep the pH, temperature and/or O₂ content constant or within a desired range. In this respect, the fermented vessel may have pH, temperature and/or O₂ content sensors and/or regulators.

The pH of the aqueous liquid during fermentation may be from 2 to 8, such as from 3 to 7, optimally from 4 to 6.

The temperature of the aqueous liquid during fermentation may be from 20 to 40°C, such as from 25 to 35°C, optimally from 27 to 33°C.

The vessel may additionally be adapted to perform, or allow to be conducted, aeration and/or agitation of the solution. The former may provide the latter, e.g. by bubbling air into the medium. Agitation may comprise stirring or other mechanical means. This may provide the mixing of the liquid and cells.

It is important that during fermentation mixing occurs. In other words, the aqueous liquid and fungal cells are suitably either mixed (together) or agitated, for example the liquid and cells are not left still. This may be achieved by aeration bubbling air into the aqueous liquid. This may serve the additional purpose of providing oxygen to the fungal cells: hence the fermentation is preferably an aerobic one.

Other means of agitation or mixing include stirring, for example using an impeller. This may be of a hydrofoil axial flow design or may be designed so that the aqueous medium is forced radially outwards from the impeller (such as a turbine). Even if there is no stirring it is preferred that the fungi are provided with oxygen during fermentation, and so aeration (e.g. by bubbling air, O₂ or other oxygen-containing gas) is advantageous here. Aeration may be at from 0.1 to 2.0, such as from 0.5 to 1.0 vvm.

One of the advantages of aeration and/or agitation is that the oxygen content of the aqueous liquid can be kept relatively high. This may be at least 10%, such as at least 15%, optimally at least 20% (in terms of air saturation). This allows a more efficient formation process, and can thus result in a quicker and/or higher content of fungal cells and/or fungal protein. The fungal cells used in the invention are sufficiently robust to allow agitation and/or mixing during fermentation. Thus with most *Mucorales* organisms one does not have to use expensive equipment, such as airlift fermentors, developed for other (less robust) organisms, which means the edible substance can be produced more cheaply.

The fermentation may take from 1 to 12 days, such as from 2 to 6 or 7 to 10 days, and optimally from 2 to 4 days. A shorter fermentation lends itself towards a batch, rather than continuous, fermentation process.

### Inherent RNA reduction by the fungal cells themselves (ripening)

This stage can be performed after stage (a) or before stage (c), but it is preferably performed before stage (b). Thus this preferably takes place while fungal cells are still inside the fermenter. Preferably fermentation will have stopped first, or the fermentation process will have been terminated. This may occur when no more N or C sources are supplied or when the (assimilable) N and/or C sources have been used up (i.e. no more assimilable and/or N sources remain in the aqueous liquid). For example, the fungal cells will have stopped growing. No liquid may be removed at this stage; the cells may thus remain in the aqueous liquid used in fermentation.

During this stage enzymes inside the fungal cells will be allowed to produce the RNA content of the fungal cells. These enzymes are usually RNAses (or ribonucleases). The temperature of the fungal cells at this stage is below 40°C, as it has been found that higher temperatures are not required in order to either activate, or keep active, the RNAses. If the cells are kept inside the fermenter vessel then the conditions can be similar to those during fermentation, in particular with regard to temperature and pH. However, the cells are held or allowed to ripen for a while after fermentation has finished in order to allow RNA degradation. In order to promote this the rate of aeration may be reduced, for example to below 0.5 vvm, such as below 0.2 vvm, for example from .05 to 0.15 vvm. The fungal cells need less oxygen during ripening as they have stopped growing. Indeed they may have entered a "starvation" phase. Mixing and/or agitation is still preferable so that the cells do not settle to the bottom of the vessel, and one may keep a homogeneous mixture. This is preferably achieved using aeration.

Thus these conditions allow a "holding" time which allows the intracellular RNAses to degrade the RNA inside the cell. This process is called ripening of the cells, and in effect it is the provision of an environment that allows the fungal cells' own RNAses to reduce the RNA intracellular content without the need for a heating (or other RNA reduction) step.

Preferably these conditions (ripening) are at a temperature of from 20-40°C, such as from 30-40°C, optimally from 33 to 37°C or 35-40°C. The pH is suitably from 3-7, such as from 4-6, optimally from 4.5-5.0. The time period (for ripening) may be from half an hour to six hours, such as 1-4 hours, optimally from 2-3 or 2½ to 3½ hours. This preferably takes place while the fungal cells are kept or remain inside the fermenter vessel.

The fungal cells after ripening preferably have an RNA content of less than 2%, such as from 0.1 to 2.0%, preferably from 0.5 to 1.5% (based on the dry weight of the cells). Optimally the RNA content is from 0.4 to 0.8%. This content may be inherent, resulting from fermentation, or the content may have been reduced (from a higher amount) as a result of ripening. Note that the RNA percentages in the specification are always based on dry matter measurements, as this is the legal requirement.

Thus this RNA reduction stage does not involve additional heat treatment (indeed the temperature is kept below 40°C) and preferably does not involve any other (e.g. external) physical, chemical or enzymatic treatment to reduce the RNA content other than allowing the cells themselves to reduce their own internal RNA content. This has the advantage that the process of the present invention can be conducted more cheaply because heating or other treatment of the cells to reduce the RNA content can be avoided.

In prior art processes the cells are often quickly removed from a fermenter and heated so as to inactivate undesirable enzymes (e.g. proteases). During growth (i.e. fermentation) there is a (dynamic) balance between RNA synthesis (in order to produce protein) and RNA degradation by RNAses. The ripening provided in the invention allows growth (and so RNA synthesis) to slow or be reduced, allowing the RNAses to reduce the RNA content faster than it is synthesised. Some protein degradation (by proteases) may thus be tolerated in return for a greater benefit, namely RNA reduction.

Thus the ripening stage, after (a) and before (b), can comprise conditioning the cells (e.g. after the end of fermentaiton, or in a starvation phase) to synthesise less RNA or to shift the balance between RNA synthesis and reduction to the latter. By using the cells own RNAses one may be able to avoid the need to force nucleic acid out of the cells, either by heat, acid or alkali treatment. There may thus be no need to make the cells more porous. Preferably the liquid is devoid of a lower alcohol, e.g. a C₁₋₄ alcohol. Suitably therefore RNA reduction is by use of intracellular enzymes (e.g. RNAses) rather than using chemical hydrolysis (with extracellular chemicals).

### Solid/liquid separation - Stage (b)

After the inherent RNA reduction, liquid (e.g. water) can be removed from the combination of the fungal cells and the surrounding liquid produced. In the art this combination of aqueous liquid and fungal cells is often referred to as a "broth". During fermentation the vessel should contain only this broth, and this is preferably entirely liquid (and so devoid of any solid material). The cells may be rinsed, such as with an aqueous liquid e.g. water, before or after this water removal stage, and either or both may result in the separation of the cells from extracellular matter (e.g. protein) if desired. If necessary depelleting (the dispersion or minimisation of any pellets in the liquid) may be conducted before or after water removal (for example by sonication or shear mixing).

Liquid (or water) removal is preferably by mechanical means or by mechanical techniques. These include various solid-liquid separation techniques such as mechanical de-watering, filtration, centrifugation (preferred), settling (in other words, the material is allowed to settle, thus using gravity) or drying. Such techniques may also be used to effect separation of (at least some of) the cells from the (aqueous) liquid.

After stage (b), or de-watering, the water content can be from 50 to 90%, such as 60 to 87%, optimally from 75 to 85%.

The protein produced may be located in various parts of the fungal cell. It may represent up to 30%, such as up to 40% and optimally up to 50% of the fungal cell itself (based on dry weight). The fungal protein may be inside the cell (intracellular) or inside the cell wall. The former may include two different "types" of proteins, for example structural proteins (those concerned with DNA; ribosomes; membranes etc) and catalytic proteins (for example enzymes). Cell wall proteins include not only those that are inside or part of the cell wall, but may be outside of the cell wall but still bound to the cell wall. This is contrast to secreted (e.g. extracellular) proteins that are not bound to the cell, and which are usually discarded or otherwise lost during processing. The material containing the fungal cells may then be subjected, if necessary, to a (further) water removal step, or de-watering. This will preferably reduce the water content to from 50 to 90%, such as from 60 to 85%, optimally from 75 to 85%. This may be after one or more rinsing or washing steps (for example with water, e.g. tap water). The resulting material may have a dry matter content of from 10 to 40%, preferably from 15 to 35%, optimally from 20 to 30%.

The liquid removed at this stage preferably contains any (e.g. RNA degradation) products or other undesired substances either removed from the cells or transferred from the inside the outside of the cells. Procedures for removing the water here are the same as described for the optional removal step earlier following fermentation. However, at this stage filtration is preferred, such as vacuum filtration.

At this stage in the process one can have produced an (e.g. aqueous) paste, a biomass or a filter cake which may have been milled, tumbled and/or centrifuged. Further processing, in particular texturising, for example using mechanical methods, can then be performed in order to produce the edible product of the second aspect.

### Killing and/or processing - Stage (c)

One may add or mix in one or more edible component(s) at this stage. These may be to add texture and/or flavour. This may be before or after mechanical processing step(s) such as milling, crumbling, cutting, kneading and/or homogenising.

Preferred components include hydrocolloids, for example pectin, starch, carrageenan or alginate. Also contemplated are proteins, for example milk protein such as casein, ovoprotein such as egg albumin or eggs themselves (yolk and/or egg white), vegetable proteins such as soy, or cereal proteins, such as gluten, or enzymes (e.g. proteases, phosphodiesterases).

Other edible components include flavour enhancers such as salt, sugar, IMP and/or GMP (although in this case it will be preferred that the RNA level does not exceed those mentioned earlier for the fungal cells), flavouring agents such as spices, herbs, proteins (e.g. from 2 to 5% such as a milk protein, e.g. casein, a vegetable protein, an ovoprotein, e.g. albumin), hydrocolloids (e.g. from 5 to 20% such as pectin, carageenan, agar, xanthan, gellan, galacturonic or mannuronic acid or salts thereof), flour, alginate (such as 0.2 to 1.0%), edible polymers (e.g. cellulose, methylcellulose) , gelling agents (such as egg albumin, whey protein and alginate), polysaccharides (such as from 0 to 10%, for example starch or pectin), colouring agents, plant material such as vegetables (onions, carrots, soy, peas, beans or cereals such as wheat, oats, barley) and emulsifiers. It may also include meat-like flavourings, such as beef, pork or poultry (chicken or turkey) flavourings or other non-meat products. Additional components may be provided to improve taste (organoleptic properties) to improve water binding, fat binding, emulsification properties, texture, volume, viscosity, flavour, aroma and/or colour (dyes, carotenoids, etc.). Egg albumin may be included to improve whippability, colouring or as a binder of other proteins. Egg yolk can be used for emulsification, colour or flavour. Soy protein can be employed for water binding, fat binding and to improve texture. Gelatin can be included to improve gelation. Milk protein or salts thereof for water binding and fat binding flavour or texture and wheat gluten for water binding, texture or flavour. The edible product may therefore be used to replace or be provided in addition to one or more of the following: vegetable proteins, egg white, gelatin, edible proteinaceous foaming agents and milk proteins. Fibrous materials may also be included (e.g. vegetables such as onions).

The processing may comprise texturization, for example to provide texture in the eventual product so that it has meat-like texture and/or it has a mouthfeel similar to meat. It may thus have a fibrous or meat-like appearance.

Texturization is preferably by one or more mechanical means. These include milling, crumbling, mincing, slicing, cutting (e.g. into chunks, slices or layers), kneading, layering, shaping, rolling, sheeting and/or extruding. Preferably it may result in the alignment of the fungal protein into fibres, which may assist to give the product the appearance of meat.

The texturising may however comprise physical methods, for example heating and/or freezing. Both of these techniques may also result in further water removal, although if steam heating is employed water may be added. Freezing in particular may assist in alignment of the fungal cells into a fibrous appearance.

The (mechanical) shaping may include placing the mixture of fungal cells and edible component(s) into a mould or other container of a desired shape, and then cooling (such as freezing) and/or heating (for example 70 to 100°C, to cause gelling, for example) by various methods such as steaming, boiling and/or frying. Pressure may be applied if necessary. The material can then be removed from the mould or container, and can retain the shape of that container.

A particularly preferred texturization method involves granulation, for example to produce granular particles. Before any texturization, the combined fungal cells and fungal protein may have an average water content of from 15 to 85%. After texturing (e.g. granulation), the resulting granules may have an average water content of below 30%, e.g. less than 20%, optionally less than 10%.

Preferably granulation is achieved using extrusion. This is preferred because extrusion conditions can be adjusted to minimise disruption of the fungal cells. Extrusion may therefore be conducted without heating, for example at from 15 to 85°C. During extrusion the granules may form naturally, falling away under their own weight (from the die plate, such as by gravity) or one can use a cutter, such as a rotating blade, to cut the long strands of "spaghetti" produced by the extrusion. Following extrusion the granules preferably have a water content less than 15%, such as less than 10%, and optimally from 3 to 7%. The granules may have a diameter of from 0.3 to 10mm, such as from 0.7 to 5mm, optimally from 1 to 3mm.

Extrusion may thus be used to form elongate "spaghetti" like products (these may be cylindrical and/or of circular cross-section) if passed through a suitable die-plate (e,g, with circular or square holes). However formation into for example sheets or layers can be achieved by passage (e.g. using extrusion) through one or more slots. These forms may also be prepared by the use of one or more moving surfaces, such as roller(s) and/or cylinder(s). These may be moving in the same direction or counter-rotating and there may be one, two or up to five such surfaces.

The product may therefore be in a variety of forms. For example, it may be in the form of chunks, for example meat-like chunks, dough, sheets, granules, extrudate, slices or may be layered. These forms may be dried or frozen. The product may be included into the foodstuff with no or additional processing. They may be recognizable as chunks in the foodstuff, and may have the appearance of meat.

They can thus be included in foodstuffs as meat substitutes, and foodstuffs contemplated include pies, microwaveable meals, savoury snacks, sausages, patties, burgers, spreads and pâté, dried powder (e.g. for soups).

The product may for example be in the shape of animals, birds or fish, letters of the alphabet, numbers, etc which may be particularly suitable for foodstuffs for children.

The product may also be in the form of a dried powder, which may be included in a foodstuff to increase mouthfeel or to increase viscosity.

The product may be in the form of pellets or granules, and these too may be dried or frozen. They may be adapted for dehydration before consumption. These products may be included in soups or sauces. The product (e.g. pellets or granules) may be included in burgers or sausages for example with an (e.g. edible) binder. A suitable sausage preparation process and sausage making machine is described in the International patent publication no. WO-A-99/55165.

It is necessary to kill the fungal cells before the foodstuff can be consumed. This (preferably single) killing step can take place at one of a number of different points in the process of the invention, and may be before, after or even during the processing of the fungal cells into the edible product of the second aspect. Thus for example cells can be killed after one or more edible components have been admixed with the cells. In this case killing may be by heating, because here the heating may serve a dual purpose and may be used to gel one or more of the components (for example if one of the components comprises a protein). However, killing may occur after the edible product has been produced, and may take place when the product is being formed or incorporated into the foodstuff in accordance with the process of the third aspect.

Killing may be achieved by chemical or physical treatment. If chemical, the cells may be contacted with a toxic substance, for example benzoic acid. The benzoic acid may be provided at a concentration of from 0.1 to 10 g/l, such as 0.5 to 5 g/l, preferably from 0.75 to 2 g/l. If physical treatment is used, then freezing is one possibility although heating (for example 70 to 100°C) is preferred (e.g. pasteurisation).

Alternatively killing can be achieved at lower temperatures, for example if one uses heating by steam then the fungal cells may be heated only to a temperature of from 40 to 65°C, preferably from 45 to 60°C, optimally from 50 to 55°C.

### Overall preferred process

Thus a particularly preferred process of the present invention may therefore comprise:
1. fermenting fungal cells of the order *Mucorales*, for example in an aqueous liquid contained in a fermenter vessel, the liquid comprising assimilable nitrogen and carbon sources. The liquid and cells can be mixed and/or aerated during fermentation and if necessary depelleting can be performed. Fermentation may be allowed to finish (or terminate) once either (the assimilable) carbon and/or nitrogen sources have been consumed. The cells may remain in the vessel for;
2. ripening the fungal cells, for example by subjecting the cells to conditions that allow RNAses inside the fungal cells to reduce the RNA content inside the cell, for example while the fungal cells remain inside the fermenter vessel (for example by allowing fermentation to finish, but continue mixing, so that the internal balance in the cells between RNA synthesis and degradation is shifted towards the latter);
3. optionally removing liquid (e.g. water), for example removing the, or water from, the aqueous liquid, preferably using mechanical techniques such as filtration, centrifugation (e.g. once or twice), settling and/or drying or otherwise separating cells from the liquid;
4. if necessary, (e.g. chemically) inactivating undesirable enzymes inside the fungal cells;
5. optionally, removing water (e.g. if not done in stage 3); such as to provide a biomass, filter cake or paste;
6. adding to the fungal cells one or more edible components;
7. texturising the fungal cells, e.g. either before (e.g. milling or crumbling) or after (e.g. kneading or extruding) edible component addition in stage 6, such as using mechanical processing;
8. subjecting the fungal cells to a physical treatment such as heating (e.g. boiling, steaming, frying) and/or freezing, or otherwise removing water or killing the cells;
9. optionally before or after stage 8, shaping and/or otherwise mechanically processing (if necessary) to give a shaped or textured product; and
10. including in or processing the edible product into, a foodstuff or supplementing a foodstuff with the product.

The product can be included into the foodstuff as it is, in other words it may simply be used to supplement an existing foodstuff, or it may be used in the preparation of a foodstuff. It may be heated first to generate nicer flavours or to brown the product.

Preferred foodstuffs include ready-made or convenience meals, or microwavable meals, burgers, pies, pasties, sausages and soups. The product can be used a substitute for meats such as pork, beef, poultry, game, ham, veal or even fish.

These foodstuffs are of course intended for human consumption, although foodstuffs for animals, in particular pets (such as dogs and cats), such as canned foodstuffs, or feed for farm animals (pigs, cows, sheep etc) are contemplated.

Other foods can be included as a component, for example rice and pasta.

Preferred features and characteristics of one aspect of the invention are applicable for another aspect *mutatis mutandis*.

The invention will now be described by way of example with reference to the accompanying Examples, which are provided merely for the purposes of illustration and are not to be construed as being limiting.

### EXAMPLES

### Comparative Example 1: Selection of suitable microorganisms

The microorganism needs to be food grade and the substance should contain "valuable" proteins. The essential nutrients as in meat should also preferably be present. The morphology/structure of the biomass has to be suitable to produce a mycoprotein enriched product with a "bite" and organoleptic sensation of meat-like products.

The Examples demonstrate that manufacturing fungal food from *Mucorales* fungi is feasible, and that the more "primitive" families within the *Mucorales* order can be preferable.

Advantages of *Mucorales* fungi include:
1. low or absent mycotoxin production;
2. simple and cheap biomass production: good growth to high concentrations in clear media (composed of salts, a well-defined complex N-source, and glucose or oligosaccharides);
3. down-stream processing procedures are acceptable for foodstuffs; and
4. good quality of end product.

### Flask experiments

In flask experiments various strains were tested belonging to the *Mucorales* families of *Choanephoraceae*, *Mucoraceae*, and *Mortierellaceae* to test their growth in simple and clear media.

Growth was tested in several different media including the two semi-defined media (one with yeast extract, the other with peptone):

| compound | concentration |
|---|---|
| yeast extract or peptone | 5 g/kg |
| glucose | 30 g/kg |
| potassium phosphate | 0.10 M |
| ammonium sulphate | 0.1 M |
| magnesium sulphate | 1.25 mM |
| zinc sulphate | 0.03 mM |
| manganese sulphate | 0.2 mM |
| iron chloride | 0.09 mM |
| copper sulphate | 0.03 mM |

The components were dissolved in deionized water, and sterilized for 20 minutes at 120°C: the glucose was sterilized separately. The pH after sterilization was 6.0.

The experiments were conducted in Erlenmeyer flasks (100/500ml). Inoculation took place with a suspension of spores prepared freshly by growing the strains for several days on a malt agar surface, rinsing the spores from the surface and storing them in a freezer. The flasks were incubated between 25 and 35°C for 2 to 4 days on an orbital shaker (with a 2.5 cm stroke at 250 rpm).

The following strains were tested:

| Species | Family | Source |
|---|---|---|
| *Blakeslea trispora* | *Choanephoraceae* | CBS 130.59 |
| *Gilbertella persicaria* | *Choanephoraceae* | CBS 247.59 |
| *Absidia pseudocylindrospora* | *Mucoraceae* | CBS 100.2 |
| *Phycomyces blakesleeanus* | *Mucoraceae* | CBS 226.92, NRRL 1555 |
| *Rhizopus oryzae* ^{*} | *Mucoraceae* | own isolate^{*} |
| *Mucor hiemalis* | *Mucoraceae* | CBS 242.35 |
| *Rhizomucor miehei*^{***} | *Mucoraceae* | own isolate^{*} |
| *Mucor rouxii* | *Mucoraceae* | CBS 416.77 |
| *Mortierella alpina*^{***} | *Mortierellaceae* | own isolate* |

| | | |
|---|---|---|
| ^{*} Strains are commercially available, e.g. from the CBS (Centraal Bureau voor Schimmelcultures, Delft, The Netherlands). | | |

All strains grew well within several days, usually in a mixed form of both filamentous mycelium and pellets. In all cases it was possible to obtain at least 5g biomass/litre of broth in the course of incubation, measured by filtering the biomass and weighing it after drying for 24 hours at 105°C on a preweighed filter paper. The presence of pellets was checked visually.

### Comparative Example 2: Fermentor experiments

As part of the scale-up process all the strains from Example 1 were subjected to lab scale fermentor experiments. The objective was to test them for growth in simple media, growth to high biomass concentration that allows further scale-ups and growth in a form that allows inclusion in a foodstuff.

The experimental set up was as follows, starting with inoculum preparation.

The spore suspension was prepared as described in the previous Example. With this spore suspension an inoculum culture was started, using a soy bean meal based medium to promote hyphal growth (soy flour 15g/kg, yeast extract 5g/kg, K₂HPO₄ 1g/kg and glucose. H₂O 20g/kg). The medium was sterilized for 45 minutes at 120°C in Erlenmeyer flasks at pH6. As soon as full growth had been reached the culture was transferred to a lab fermentor, containing medium that was prepared using the following components:

| component | concentration (g/kg) |
|---|---|
| yeast extract | 1 |
| glucose | 20 |
| ammonium sulphate | 6 |
| magnesium sulphate.7 H₂O | 2 |
| calcium chloride | 0.5 |
| potassium monophosphate | 3 |
| zinc sulphate.7H₂O | 0.0144 |
| iron sulphate.7H₂O | 0.15 |
| manganese sulphate. 1H₂O | 0.0228 |
| copper sulphate.5H₂O | 0.0024 |
| cobalt sulphate.7H₂O | 0.0038 |
| thiamine.HCl | 0.004 |
| nicotinic acid | 0.002 |
| | |

All compounds were dissolved in deionized water and mixed, except the glucose and the phosphate which were prepared separately. The pH was adjusted to 6.0 or 4.5 using NaOH, and the medium was sterilized in the fermentor for 45 minutes at 121°C in an autoclave. The glucose solution and phosphate solution were added after separate sterilization for 20 minutes at 120°C, the first after acidification to pH 5 with phosphoric acid.

Next to the batch medium a carbohydrate feed was supplied which consisted of glucose at a concentration of ca. 500 g/kg. The preparation was as described for the glucose solution of the batch medium.

The fermentor was equipped with temperature, pH and foam controls. To adjust the pH, solutions of ammonia and sulphuric acid were used. Dissolved oxygen concentration and the composition of the liberated gas were measured. The culture was aerated using ca. 1 volume of air per volume of broth per minute. Mixing was intensive using Rushton turbines and baffles. The glucose feed was applied at a rate between 1 and 5g of glucose/kg broth/hour and started when the glucose concentration in the broth had decreased to a concentration below 5 g/kg. The temperature during fermentation was 30-40°C (depending on the organism used), the starting pH from 4.0 to 6.0 (again, according to the strain, adjusting for optimal growth) and fermentation lasted for about 80 hours.

Samples were taken twice every 24 hours for off-line analysis of concentrations of unused substrate, biomass and by-products. Microscopic inspection was also performed.

The following strains, thus tested in flasks for experiments in lab scale fermentors, were selected:

*Rhizopus oryzae*, *Mortierella alpina*, *Gilbertella persicaria and Absidia pseudocylindrospora.*

In all cases the biomass accumulated to concentrations from 20 to 50 g/kg within 80 hours of cultivation.

All strains thus indicated potential to be able to produce biomass in a simple medium and at a low cost when scaling up the process.

### Comparative Example 3: Morphology analysis

In shake flasks various microorganisms were cultivated according to the procedures given in Example 1. The morphology of the biomass was examined by light microscopic methods. The characteristics found are shown in Table 1. The morphology of the *Mucorales* organisms was different to that of the *Fusarium* species.

**TABLE 1**

| Strain | Branched |
|---|---|
| *Fusarium graminearum* (now reclassified as *F. venenatum*, IMI 145425) | No |
| *Mortierella alpina* | Yes |
| *Gilbertella persicaria* | Yes |
| *Rhizopus oryzae* | Yes |
| *Absidia pseudocylindrospora* | Yes |

### Examples 4 and 5 and Comparative Example 6: lab scale fermentation and biomass analysis

In Example 4, lab scale fermentors were used to cultivate three microorganisms (*Absidia pseudocylindrospora*, *Gilbertella persicaria* and *Mortierella alpina*) according to the procedures described under Example 2 and the same conditions were used to culture *Fusarium venenatum* (Comparative Example 6).

In Example 5 *Rhizopus oryzae* was also cultured on a laboratory scale (for details see Example 2).

The following recovery procedure was used to first prepare a biomass filtercake:
1. directly after the end of fermentation fungal cells were allowed to ripen. The temperature of the broth (if below 35°C) was increased to 35°C and this temperature was maintained for 3 hours, while the pH was kept at 4.5. Mixing continued for this period but the aeration was lowered to 0.1 volume of air per volume of broth per minute;
2. centrifugation and washing the biomass (to remove excess medium components like glucose and salts);
3. filtration on a lab filter press, including washing with tap water; and
4. packing and storage.
Centrifugation. Portions of 1 litre of biomass were centrifuged in a Beckmann centrifuge (type J-6M/E) for 5 minutes at 5000 rpm. The supernatant was decanted and discarded. The pellet was resuspended in tap water and recentrifuged. The supernatant was decanted again. The washed pellet was resuspended in tap water.

Filtration: the heated broth was filtered in a 2 litre filter press (type Seitz Enzinger Noll, Germany) provided with a polypropylene filter cloth at a starting pressure of 0.5 bar.

The resulting cake was washed with 10 cake volumes of tap water. After washing the cake was blown dry with air at 2 bar for 15 minutes. The cake was collected for further treatment. The cake was analysed for dry matter and the RNA data resulting from analysis is given in Table 2.

**TABLE 2**

| Example | Strain | start volume (ml) | total cake (g) | dry matter of cake (%) | RNA after ripening (% on dry matter) |
|---|---|---|---|---|---|
| 4 | *Mortierella alpina* | 3250 | 282 | 26 | 1.5 |
| 4 | *Gilbertella persicaria* | 3700 | 1135 | 15.3 | 1.2 |
| 4 | *Absidia pseudocylindrospora* | 3400 | 284 | 20.6 | 1.9 |
| 5 | *Rhizopus oryzae* | 10000 | 1410 | 14.3 | 0.7 |
| 6 (Comp) | *Fusarium graminearum* | 3500 | 552 | 24.6 | 6.0 |

### Example 7: Preparation of edible biomass

Two production fermentations the *Rhizopus oryzae* strain used in Example 2 were performed in a standard production fermenter with a working volume of 30 m³. The fermenter had a pH control, a Rushton turbine with adjustable speed, air supply, foam control and temperature control. At the end of the fermentation the temperature was increased to 38°C and the cells allowed to ripen and reduce their intracellular content. The broth was then cooled to below 20°C. The broth was transferred to another vessel, diluted with cold tap water and further cooled to 4-6°C. The cooled broth was then filtered in a Schenk membrane filter press with a working cake volume of 2.5 m³ and the cake washed with 20 m³ of cold tap water. The cake was squeezed by applying pressurized water in a membrane system (4-9 bar). The cake was discharged from the filter press and part of this crumbled, packed in bags and frozen in a cold store at -15 to -20°C. Part of the frozen biomass was cut into particles having a size of from 1-3mm.

The two samples were further analysed chemically for RNA and had an RNA content of 1.9% and 1.4% (based on dry matter), respectively.

### Example 8: Dried biomass

The remaining cut particulate biomass for Example 7 was dried in portions of 30-50kg in a Aeromatic T4 fluid bed dryer with a bottom plate area of 0.26 m² with dry air at a temperature of from 55-65°C. Drying terminated at a bed temperature of 38-40°C.

### Examples 9A, 9B and 9C: Sheeting, layering, rolling

The filter cake from Example 7 was milled and crumbled in portions of approximately 25kg by a Lödige high shear mixer for 5 minutes. To the crumbled cake 1kg of egg albumin (Example 9A) was added and the mixture kneaded. The procedure was repeated with a little water and spices being first mixed with the egg albumin (Example 9B).

The mixture was formed into sheets of 1mm by rolling equipment.

The sheets were heated to 80°C in an ventilated oven or tunnel. The sheets were layered and rolled in the form of a "Swiss roll" and the roll frozen to -20°C using liquid carbon dioxide.

The same procedure was repeated except 1kg pectin (Example 9C) was substituted for the egg albumin.

### Examples 10A to D: Burgers

To the biomass from Examples 7 and 8 colouring additives, taste enhancing products (spices, vegetables and onions) were added. The mixture was then homogenised in a kneader and the homogenised mixture formed into burgers, pasteurised and packed. Both procedures were repeated with egg albumin being first mixed with the taste enhancers.

### Examples 11A to D: Sausages

To the biomass from Examples 7 and 8 colour additives, spices, vegetables (onions) were added. The mixture was homogenised in a kneader. The homogenised mixture was extruded into a continuous tube (so that it formed the interior of the sausages) while coextruding a (vegetarian) skin-forming material using a continuous sausage-making system (Stork) to make sausages. The two procedures were then repeated with egg albumin being first mixed with the colour additives and spices.

### Examples 12A and B: Granules

The filter cake from Example 7 was milled and crumbled into portions of approximately 25kg by a Lödige high shear mixer for 5 minutes. To the crumbled cake 1kg of egg albumin was added and the mixture kneaded. The kneaded mixture was extruded with a single screw extruder with a dieplate with holes of 1 mm. The extrudate was transported by a belt and dried in a fluidised bed drier (air temperature of 50°C) to form granules. For 12B pectin was used in the same amount instead of egg albumin.

### Examples 13A to D: Burgers

A batch of 25kg of the dried extrudate from each of Examples 12A and B was mixed with 60kg tap water. To this mixture the food additives used in Example 9 (egg albumin or pectin) were added and the mixture kneaded and formed into burgers, pasteurised, packed and frozen.

### Examples 14A to D: Sausages

A batch of 25kg of the dried extrudate from each of Examples 12A and 12B was mixed with 60kg tap water. To each mixture the food additives from Example 9 (egg albumin or pectin) were added and the mixture processed into sausages as described in Example 11.

### Examples 15A and B: Burgers

To biomass 25kg from each of Examples 7 and 8 was added colour additives and taste enhancing products (spices, vegetables, onions). To the mixture 1kg vegetable fibres (cellulose fibres with an average fibre length of 300-1000µm) was added and homogenised in a kneader. The homogenised mixture was formed into burgers, packed, pasteurised and frozen.

### Examples 16A and B: Sausages

To biomass (25kg) from each of Examples 7 and 8 colour additives, spices, vegetables and onions were added. To the resulting mixture 1kg vegetable fibres (cellulose fibres with an average fibre length of 300-1000µm) was added and the mixture homogenised in a kneader. The homogenised mixture was formed by extrusion into sausages by co-extrusion with a vegetarian skin as described in Example 11.

### Examples 17A and B: Granules for soups

The filter cake from Examples 7 and 8 was milled and crumbled in portions of approximately 25kg by a Lödige high shear mixer for 5 minutes. To the crumbled cake a mixture of 1kg egg albumin was added and 1kg vegetable fibres (cellulose fibres with an average fibre length of 300-1000µm). The mixture was kneaded and then extruded in a single screw extruder with a dieplate with holes of 1mm. The extrudate was transported by a belt and dried in a fluidised bed dryer (air temperature of 65 to 80°C), to form granules. These were then added to a soup and dried to form soup powder (that produces soup on rehydration).

### Examples 18A and B: Burgers

A batch of 25kg of the dried extrudate from Examples 17A and B was mixed with 60kg tap water. To the mixture the food additives described in Example 15 were added and the mixture kneaded and used to make burgers as described in Example 15.

### Examples 19A and B: Sausages

A batch of 25kg of the dried extrudate from Examples 17A and B was mixed with 60kg tap water. To the mixture the ingredients as described in Example 16 were added, the mixture kneaded and used to form sausages as described in Example 16.

### Example 20 and Comparative Example 21: Preparation of Comparative patties and organoleptic testing

Two labscale fermentor experiments were performed according to Example 2. The first fermentation was harvested and the fermentation broth was filtrated with a 2 litre pressure-filter. The resulting cake was washed 3 times with 3 litres of water at about 4-10°C. The filtercake was blown dry by air at a pressure of 1.8 bar. The filtercake (Example 20) was stored at -18°C.

The second fermentation was harvested and the fermentation broth was filtered using a 2 litre pressure-filter. The resulting cake was washed 3 times with 3 litres of water at about 4-10°C. The filtercake was resuspended in water, 1 g/l benzoic acid was added, the pH was adjusted to pH 4.0 with phosphoric acid and after a holding time of 30 minutes at 50°C (to kill the cells) the suspension was filtered and the cake was washed. The filtercake was blown dry by air and stored at -18°C (Comparative Example 20).

Patties were then made according to the following protocol. 100g of biomass (25% dry weight) was cut and mixed with 35g water, 6g egg albumin and 4.6g soy oil in a lab scale food processor (Braun Combi type 700). The resulting dough was placed in moulds and heated to 80°C by steaming. After chilling to 4-7°C the patties were frozen to -20°C.

A panel of 4 expert tasters judged blind the organoleptic properties of the two pattysamples after microwave heating. The panel judged the patty of Example 20 as much more neutral in taste than the patty of Example 21. This last sample (21) was characterised as having a bitter, unpleasant taste.

## Claims

1. A process for the preparation of an edible product comprising fungal cells, that is suitable for use in a foodstuff, the process comprising:
(a) fermenting fungal cells of the order *Mucorales* in an aqueous liquid contained in a fermenter vessel, the liquid comprising an assimilable nitrogen
(N) source and an assimilable carbon (C) source, and mixing the liquid and cells in that vessel during fermentation;
(b) separating at least some of the cells from the mixture of fungal cells and aqueous liquid or reducing the liquid content of the mixture of liquid and cells; and
(c) processing the fungal cells into an edible product;
wherein before (c) the temperature of the fungal cells is kept below 40°C and the fungal cells are subjected to conditions that allow enzymes inside the fungal cells to reduce the RNA content of the fungal cells.

2. A process according to claim 1 which excludes an (e.g. physical and/or chemical) RNA reduction step.

3. A process according to claim 1 or 2 which excludes heating the fungal cells (such as to above 40°C) to reduce their RNA content and/or chemically degrading the RNA inside the cells.

4. A process according to any preceding claim wherein the enzymes are RNAses, the RNA is intracellular and/or after (a) but before (b) the fungal cells are kept inside the vessel while the enzymes reduce the RNA content of the fungal cells (ripening).

5. A process according to any preceding claim where the RNA content of the cells is, or has been reduced to, below 2%.

6. A process according to any preceding claim wherein the fungal cells are of the genus *Rhizopus.*

7. A process according to any preceding claim wherein (c) comprises mixing one or more edible component(s) with the fungal cells, optionally mechanically texturising the resulting mixture, and if necessary killing the fungal cells.

8. An edible product, suitable for use in a foodstuff, comprising fungal cells of the order *Mucorales* that have been subjected to conditions which allows the cells to have an RNA content below 2%.

9. A product according to claim 8 of which at least 5% is fungal cells (on a dry matter weight basis) and/or which is preparable by a process according to any one of claims 1 to 7.

10. A product according to claim 8 or 9 which comprises pellets, granules, a sheet, or is an extrudate, dough, roll, paste or an (e.g. meat-like) chunk.

11. A process for the preparation of a foodstuff comprising forming a foodstuff with, or including into an existing foodstuff, an edible product according to claim 8 or 9.

12. A foodstuff which comprises an edible product according to claim 8 or 9, for example a sausage, patty, burger, spread, pâté, animal feed, tablet, pie, savoury snack or oven-ready meal.
